# EUROPEAN PATENT APPLICATION

(11) **EP 4 431 004 A1**
(43) Date of publication of application: **18.09.2024**
(21) Application number: 22884953.5
(22) Date of filing: 23.03.2022
(51) Int. Cl.: A61B 1/00, A61M 25/00

(54) **DISPOSABLE SOFT-ENDOSCOPE CATHETER AND PREPARATION METHOD THEREFOR**

(30) Priority: 01.11.2021 CN 202111284948
(71) Applicant: Nantong Enove Precision Plastics Catheter Co., Ltd., Nantong, Jiangsu 226004 (CN)
(72) Inventor: QIN, Xiaopeng, Nantong, Jiangsu 226004 (CN); WEI, Xianfeng, Nantong, Jiangsu 226004 (CN)
(74) Representative: Patent 42
(86) International application number: PCT/CN2022/082597
(87) International publication number: WO 2023/071036

(57) **Abstract**

A disposable soft scope catheter includes a hard tube end and a soft tube end. The hard tube end includes an inner-layer hard tube, a toughened structural layer, and an outer-layer hard tube provided with the toughened structural layer on an inner surface. A hard tube portion of the soft scope catheter is fabricated first, such that a woven steel wire mesh of a hard tube segment extends to a length required by the entire soft scope catheter. The soft tube end employs a two-layer structure, thereby facilitating one-step nesting of a snake bone structure inside a front end of the soft tube. A step formed between the steel wire mesh and the inner tube layer of the hard tube segment inside the steel wire mesh may be used for nesting, limiting and fixing a snake bone joint.

## Description

### Technical Field

The present invention relates to the technical field of medical care catheters, and particularly to a disposable soft scope catheter and a fabrication method thereof.

### Background Art

Soft scopes, as the name suggests, are flexible and bendable scopes. In medicine, scopes used for different systems are classified into many types, such as laryngopharyngeoscopes for the otorhinolaryngologic system, bronchofiberscopes for the respiratory system, choledochoscopes and colonoscopes for the digestive system, and multiple types of scopes for the urinary system, such as soft ureteroscopes, nephroscopes, soft nephroscopes, and soft cystoscopes. These scopes all belong to the soft scope series, and can be classified into two general categories in terms of structures and constructions, one category including fibrous soft scopes and the other category including electronic soft scopes. The soft scopes for the urinary system are highlighted here. The soft scopes for the urinary system include soft nephroscopes which are mainly used for removal and crushing of kidney stone, especially stone in the minor renal calices, as the soft scopes are bendable. In addition, the soft ureteroscopes are typically used for the treatment of upper urinary tract stone, including stone above the ureters. The soft cystoscopes are mainly used for urethrocystoscopy. Compared with the hardware of urethrocystoscopes, the soft cystoscopes cause less pain to patients during an examination process and can achieve the effect of scoping bending sites of the urethra and bladder due to the bendable property thereof.

Defects in the existing technology:
The conventional disposable soft scopes are generally manufactured by combining soft and hard segments, so as to ensure the sealing performance and electronic shielding function of an entire insertion portion while bending is achieved. In order for bending, a snake bone joint needs to be implanted into the front end of the soft scope. However, in the conventional process, a hard segment portion of a soft scope catheter is fabricated first, then a snake bone is nested therein, and the snake bone butts against a distal end port of a hard tube. Next, the snake bone sleeves a woven steel wire mesh, then two ends of the steel wire mesh which the snake bone sleeves are spot-welded and fixed to two ends of the snake bone, and finally the steel wire mesh of a soft segment portion of the soft scope catheter is wrapped with a soft rubber tube, with a distal end socket of an insertion tube sleeving a proximal end at 1 cm and a module sleeving a distal end. Sealing is achieved by glue adhesion. The conventional process achieves fixing of the snake bone ends and distinguishing between soft and hard materials of the external catheter of the soft scope, and achieves segmental connection and fixation of the snake bone and a lens. Such a fabrication process has a simple principle but has complex procedures. Moreover, an additional steel wire mesh welding procedure is required at a position for fixing the snake bone, thereby having requirements for the welding quality and having the possibility of damaging the snake bone and hard segment ends. In the subsequent wrapping with the soft tube, it is also easy to lead to the problems such as a protruding joint at a junction of the soft and hard segments, the presence of a step at the catheter, unsmooth transition of the catheter, and less aging resistance of the glue adhesion, which affects the use feeling of patients and product quality.

### Summary

The purpose of the present invention is to provide a disposable soft scope catheter and a fabrication method thereof, so as to solve the problems as proposed in the background.

To achieve the above purpose, the present invention provides the following technical solution: a disposable soft scope catheter includes a hard tube end and a soft tube end. The hard tube end includes an inner-layer hard tube, a toughened structural layer, and an outer-layer hard tube. The toughened structural layer is disposed on an inner surface of the outer-layer hard tube, and the inner-layer hard tube is disposed on an inner surface of the toughened structural layer.

The toughened structural layer is either of a woven mesh structural layer and a spiral spring.

The soft tube end includes an outer-layer soft tube that is disposed at one end of the outer-layer hard tube, and a snake bone structure is disposed inside the outer-layer soft tube and is disposed on an inner side wall of the toughened structural layer.

As a further improvement of the present invention, a hardness of the inner-layer hard tube is greater than a hardness of the outer-layer hard tube, the toughened structural layer extends to an end of the outer-layer soft tube away from the outer-layer hard tube, and a step structure is disposed at an end of the inner-layer hard tube close to the outer-layer soft tube.

As a further improvement of the present invention, the snake bone structure includes several snake bones, one end of the snake bone structure is disposed on an end face of the step structure of the inner-layer hard tube, a thickness of the snake bone structure is less than a tube wall thickness of the inner-layer hard tube, and an upper bending degree and a lower bending degree of the snake bone structure are both greater than 280°.

As a further improvement of the present invention, a linking structure of the snake bone structure is molded integrally by laser-cutting a metal round tube, and the linking structure of the snake bone structure includes a patterned rotation structure that includes first-stage rotation members and second-stage rotation members fitting with the first-stage rotation members, wherein the first-stage rotation members include first-stage arc-shaped slide blocks and second-stage arc-shaped slideways, and the second-stage rotation members include first-stage arc-shaped slideways and second-stage arc-shaped slide blocks. The first-stage arc-shaped slide blocks fit with the first-stage arc-shaped slideways, the second-stage arc-shaped slide blocks fit with the second-stage arc-shaped slideways, and the first-stage arc-shaped slide blocks, the second-stage arc-shaped slideways, the first-stage arc-shaped slideways, and the second-stage arc-shaped slide blocks are concentric arcs.

As a further improvement of the present invention, in the patterned rotation structure, the second-stage rotation members fitting with two ends of the first-stage rotation members are disposed vertically. When the metal round tube is in a straight line state, ends of the first-stage arc-shaped slide blocks and ends of the first-stage arc-shaped slideways form first-stage rotation avoidance clearances and third-stage rotation avoidance clearances, ends of the second-stage arc-shaped slide blocks and ends of the second-stage arc-shaped slideways form second-stage rotation avoidance clearances and fourth-stage rotation avoidance clearances, and the first-stage rotation avoidance clearances and the second-stage rotation avoidance clearances are disposed symmetrically with respect to both an X-axis and a Y-axis of a center of the concentric arcs.

The first-stage arc-shaped slide blocks perform an arc reciprocating motion along the first-stage arc-shaped slideways under the action of an external force, while the second-stage arc-shaped slide blocks perform an arc reciprocating motion along the second-stage arc-shaped slideways, such that the first-stage rotation members and the second-stage rotation members form a relative bending state from an initial position.

As a further improvement of the present invention, the woven mesh structural layer includes any one of nylon, a metal, a fiber, and fluoroplastic.

The present invention further provides a fabrication method of a disposable soft scope catheter. The fabrication method of the disposable soft scope catheter in the present invention includes the following steps: S1: inner-layer hard tube fabrication; S2: toughened structural layer application; S3: outer tube combination; and S4: snake bone structure nesting, wherein:
S1: inner-layer hard tube fabrication includes: first fabricating the inner-layer hard tube of a hard segment of the soft scope catheter through once molding, and performing deburring treatment on two ends and outer and inner side surfaces of the inner-layer hard tube;
S2: toughened structural layer application includes: arranging the toughened structural layer on an outer surface of the inner-layer hard tube obtained in step S1: inner-layer hard tube fabrication, with a length of the toughened structural layer being greater than a length of the inner-layer hard tube, such that a step is formed inside the hard segment of the catheter;
S3: outer tube combination includes: fabricating an outer-layer tube at once by combining the outer-layer hard tube and the outer-layer soft tube, and adhering the outer-layer tube entirely in a wrapping manner to an outer surface of the toughened structural layer obtained in step S2: toughened structural layer application; and
S4: snake bone structure nesting includes: nesting the snake bone structure on the inner side wall of the toughened structural layer inside the outer-layer soft tube obtained in step S3: outer tube combination, and abutting one end of the snake bone structure against the step of the inner-layer hard tube.

As a further improvement of the present invention, for the inner-layer hard tube obtained in step S1: inner-layer hard tube fabrication, prior to step S2: toughened structural layer application, abrasion treatment needs to be performed on the outer surface of the inner-layer hard tube, while polishing treatment is performed on the inner side surface of the inner-layer hard tube.

As a further improvement of the present invention, the length of the toughened structural layer obtained in step S2: toughened structural layer application is greater than the length of the inner-layer hard tube, and the length of the toughened structural layer is the same as a length of the outer-layer tube obtained in step S3: outer tube combination.

The present invention provides a disposable soft scope catheter further including a flexible joint driving device. The flexible joint driving device includes an operation handle housing, traction wires, a limiting buffer component, and a rear end control mechanism, wherein the traction wires, the limiting buffer component, and the rear end control mechanism are all disposed inside the operation handle housing, one end of each of the traction wires passes through the limiting buffer component and is connected to the rear end control mechanism, and the other end of each of the traction wires is connected to the snake bone structure.

Compared with the existing technology, the present invention has the following beneficial effects:
1. In the present invention, the soft scope catheter is designed to be a layered structure. A hard tube segment has a three-layer structure, wherein an inner tube layer has the highest hardness, an outer tube layer has the second highest hardness, and a woven steel wire mesh is nested between the two layers. A front-end soft tube segment has a two-layer structure including no inner tube layer, thereby facilitating one-step nesting of the snake bone structure inside a front end of the soft tube. Moreover, the step formed between the steel wire mesh and the inner tube layer of the hard tube segment inside the steel wire mesh may be used for nesting, limiting and fixing a snake bone joint, thereby effectively improving reliability and flexibility of the snake bone.
2. In the present invention, through the once molding fabrication process of the soft scope catheter, a hard tube portion of the soft scope catheter is fabricated first, such that the woven steel wire mesh of the hard tube segment extends to a length required by the entire soft scope catheter, thereby saving snake bone nesting and spot-welding procedures in an early phase of a conventional process. The integral woven steel wire mesh improves a structural strength of the soft scope catheter greatly.
3. In the present invention, the outer-layer tube can be fabricated by directly adhering and fixing the outer-layer hard tube and the outer-layer soft tube or fabricated by means of a gradual soft-hard change, thereby avoiding instability of segmental fixation followed by adhesion in the conventional process, while improving smoothness of an outer-layer surface of the soft scope catheter, and ensuring use comfort of patients during surgeries.
4. In the present invention, the linking structure of the snake bone molded integrally by laser cutting may ensure that the snake bone moves more flexibly and effortlessly in a wide motion range. A snake bone having a diameter less than 3 mm may be produced through the laser cutting, thereby solving the problem that the existing snake bone can only be achieved through riveting. As such, the snake bone is easy to process, the product consistency is improved to facilitate subsequent installation, a yield of the snake bones may be increased, the production costs of the snake bones are reduced, and axial loosening at both ends of the patterned rotation structure is avoided. By configuring the first-stage arc-shaped slide blocks, the second-stage arc-shaped slideways, the first-stage arc-shaped slideways, and the second-stage arc-shaped slide blocks as concentric arcs, an inner circle (i.e., a connection and fit relationship between the first-stage arc-shaped slideways and the first-stage arc-shaped slide blocks) is used to ensure a unidirectional rotation motion of the patterned rotation structure, and an outer circle (i.e., a connection and fit relationship between the second-stage arc-shaped slideways and the second-stage arc-shaped slide blocks) is used to ensure that both ends of the patterned rotation structure do not loosen axially. The provision of the third-stage rotation avoidance clearances and the fourth-stage rotation avoidance clearances may cooperate with the inner circle and the outer circle to avoid the axial loosening of the patterned rotation structure. Meanwhile, the first-stage rotation avoidance clearances and the second-stage rotation avoidance clearances are disposed symmetrically with respect to both the X-axis and the Y-axis of the center of the concentric arcs, so as to reduce or avoid patterned rotation.
5. In the present invention, in order to facilitate installation carried out by workers, a hook hinge is used to control a soft lock to drive a motion of the soft scope catheter with good stability. Soft lock driving may enable the soft scope catheter to produce a multi-degree of freedom motion, thereby increasing flexibility of the soft scope catheter and satisfying different application situations of the soft scope catheter. The soft lock driving is divided into three major portions. The limiting buffer component realizes an adjustment of each traction wire, thereby ensuring consistency in tensile forces of all the traction wires while protecting the traction wires. Due to different rear end structure designs, there is a risk of entanglement and breakage of the traction wires during a motion and a process of reaching the rear end control mechanism. In this case, guide tubes made of guide spring tubes or guide soft tubes may be selected to protect the traction wires, thereby increasing stability during the motion and a service life of the traction wires. Meanwhile, the limiting buffer component also plays a role of guiding the traction wires. The guide tubes guide each traction wire to reach a desired position and to be connected to the rear end control mechanism, so as to ensure a stable state of each traction wire during the driving process, thereby solving the problem of entanglement in the soft lock.

### Description of the Drawings

FIG. 1 is a schematic diagram of an overall structure of a disposable soft scope catheter of the present invention;
FIG. 2 is a fabrication flowchart of a fabrication method of a disposable soft scope catheter of the present invention;
FIG. 3 is a schematic diagram of a straight line state of a snake bone structure of a disposable soft scope catheter of the present invention;
FIG. 4 is a schematic diagram of a bending line state of a snake bone structure of a disposable soft scope catheter of the present invention;
FIG. 5 is a partial enlarged diagram of a snake bone structure of a disposable soft scope catheter of the present invention;
FIG. 6 is a connection diagram of a soft scope catheter and a flexible joint driving device of a disposable soft scope catheter of the present invention; and
FIG. 7 is a schematic diagram of structural fit of a first fixing member, a first locking structure and a spring member of a disposable soft scope catheter of the present invention.

In the figures: 1. hard tube end; 101. inner-layer hard tube; 102. toughened structural layer; 103. outer-layer hard tube; 2. soft tube end; 21. outer-layer soft tube; 3. snake bone structure; 31. patterned rotation structure; 3101. first-stage arc-shaped slide block; 3102. second-stage arc-shaped slideway; 3103. first-stage arc-shaped slideway; 3104. second-stage arc-shaped slide block; 32. first-stage rotation avoidance clearance; 33. third-stage rotation avoidance clearance; 34. second-stage rotation avoidance clearance; 35 fourth-stage rotation avoidance clearance; 4. flexible joint driving device; 41. operation handle housing; 42. traction wire; 43. limiting buffer component; 431. first fixed seat; 432. first clamping slot; 433. buffer spring; 434. second fixed seat; 435. second clamping slot; 436. guide tube; 437. threaded sleeve tube; 438. adjusting bolt; 44. rear end control mechanism.

### Embodiments

The technical solution in embodiments of the present invention will be clearly and completely described below in conjunction with the drawings in the embodiments of the present invention. Apparently, the described embodiments are only part, but not all, of the embodiments of the present invention. Based on the embodiments in the present invention, all other embodiments obtained by those of ordinary skills in the art without the exercise of inventive effort fall within the scope of protection of the present invention.

### Embodiment 1

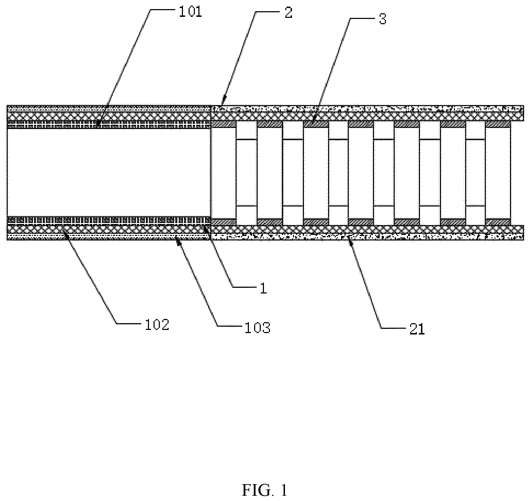

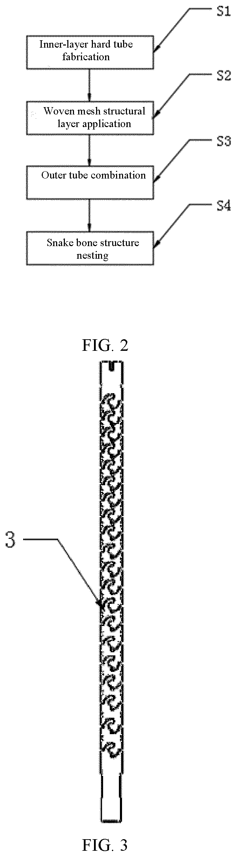

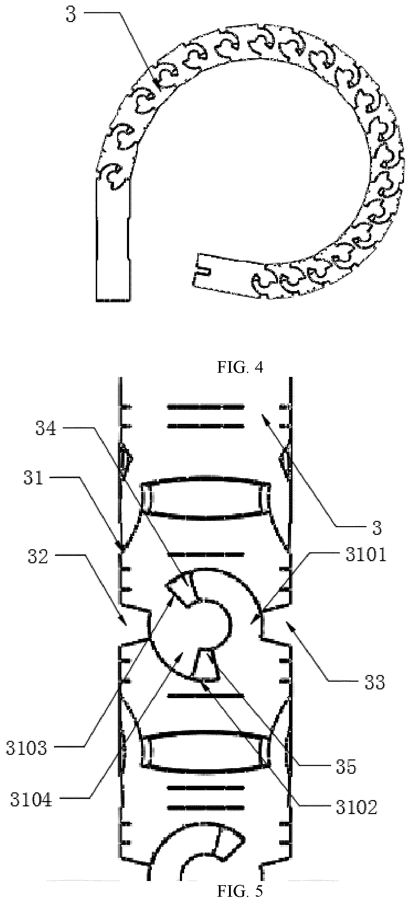

Referring to FIGs. 1-5, the present invention provides the following technical solution: a disposable soft scope catheter includes a hard tube end 1 and a soft tube end 2. The hard tube end 1 includes an inner-layer hard tube 101, a toughened structural layer 102, and an outer-layer hard tube 103. The toughened structural layer 102 is disposed on an inner surface of the outer-layer hard tube 103, and the inner-layer hard tube 101 is disposed on an inner surface of the toughened structural layer 102.

The toughened structural layer 102 is either of a woven mesh structural layer and a spiral spring.

The soft tube end 2 includes an outer-layer soft tube 21 that is disposed at one end of the outer-layer hard tube 103. A snake bone structure 3 is distributed inside the outer-layer soft tube 21, and nested on an inner side wall of the toughened structural layer 102.

Preferably, a hardness of the inner-layer hard tube 101 is greater than a hardness of the outer-layer hard tube 103, the toughened structural layer 102 extends to an end of the outer-layer soft tube 21 away from the outer-layer hard tube 103, and a step structure is formed at an end of the inner-layer hard tube 101 close to the outer-layer soft tube 21.

Preferably, the snake bone structure 3 includes several snake bones. One end of the snake bone structure 3 abuts against an end face of the step structure of the inner-layer hard tube 101. A thickness of the snake bone structure 3 is less than a tube wall thickness of the inner-layer hard tube 101. An upper bending degree and a lower bending degree of the snake bone structure 3 are both greater than 280°.

Preferably, a linking structure of the snake bone structure 3 is molded integrally by laser-cutting a metal round tube, and includes a patterned rotation structure 31. The patterned rotation structure 31 includes first-stage rotation members and second-stage rotation members fitting with the first-stage rotation members, wherein the first-stage rotation members include first-stage arc-shaped slide blocks 3101 and second-stage arc-shaped slideways 3102, and the second-stage rotation members include first-stage arc-shaped slideways 3103 and second-stage arc-shaped slide blocks 3104. The first-stage arc-shaped slide blocks 3101 fit with the first-stage arc-shaped slideways 3103, the second-stage arc-shaped slide blocks 3104 fit with the second-stage arc-shaped slideways 3102, and the first-stage arc-shaped slide blocks 3101, the second-stage arc-shaped slideways 3102, the first-stage arc-shaped slideways 3103, and the second-stage arc-shaped slide blocks 3104 are concentric arcs.

Preferably, in the patterned rotation structure 31, the second-stage rotation members fitting with two ends of the first-stage rotation members are disposed vertically. When the metal round tube is in a straight line state, ends of the first-stage arc-shaped slide blocks 3101 and ends of the first-stage arc-shaped slideways 3103 form first-stage rotation avoidance clearances 32 and third-stage rotation avoidance clearances 33. Ends of the second-stage arc-shaped slide blocks 3104 and ends of the second-stage arc-shaped slideways 3102 form second-stage rotation avoidance clearances 34 and fourth-stage rotation avoidance clearances 35. The first-stage rotation avoidance clearances 32 and the second-stage rotation avoidance clearances 34 are disposed symmetrically with respect to both an X-axis and a Y-axis of a center of the concentric arcs.

The first-stage arc-shaped slide blocks 3101 perform an arc reciprocating motion along the first-stage arc-shaped slideways 3103 under the action of an external force, while the second-stage arc-shaped slide blocks 3104 perform an arc reciprocating motion along the second-stage arc-shaped slideways 3102, such that the first-stage rotation members and the second-stage rotation members form a relative bending state from an initial position.

The linking structure of the snake bone molded integrally by laser cutting solves the problem that the existing snake bone can only be achieved through riveting. As such, the snake bone is easy to process, and the product consistency is improved.

Preferably, the woven mesh structural layer includes any one of nylon, a metal, a fiber, and fluoroplastic.

The present invention further provides a fabrication method of a disposable soft scope catheter. The fabrication method of the disposable soft scope catheter in the present invention includes the following steps: S1: inner-layer hard tube fabrication; S2: toughened structural layer application; S3: outer tube combination; and S4: snake bone structure nesting, wherein:
S1: inner-layer hard tube fabrication includes: first fabricating the inner-layer hard tube 101 of a hard segment of the soft scope catheter by once molding, and performing a deburring treatment on two ends and outer and inner side surfaces of the inner-layer hard tube 101;
S2: toughened structural layer application includes: arranging the toughened structural layer 102 on an outer surface of the inner-layer hard tube 101 obtained in step S1: inner-layer hard tube fabrication, with a length of the toughened structural layer 102 being greater than a length of the inner-layer hard tube 101, such that a step is formed inside the hard segment of the catheter;
S3: outer tube combination includes: fabricating an outer-layer tube at once by combining the outer-layer hard tube 103 and the outer-layer soft tube 21, and adhering the outer-layer tube entirely in a wrapping manner to an outer surface of the toughened structural layer 102 obtained in step S2: toughened structural layer application; and
S4: snake bone structure nesting includes: nesting the snake bone structure 3 on the inner side wall of the toughened structural layer 102 inside the outer-layer soft tube obtained in step S3: outer tube combination, and abutting one end of the snake bone structure 3 against the step of the inner-layer hard tube 101.

Preferably, in the present invention, for the inner-layer hard tube 101 fabricated in step S1: inner-layer hard tube fabrication, prior to step S2: toughened structural layer application, abrasion treatment needs to be performed on the outer surface of the inner-layer hard tube 101, so as to ensure that the outer surface of the inner-layer hard tube 101 provides a friction force sufficient to better fix the toughened structural layer 102 on the outer surface of the inner-layer hard tube 101, while polishing treatment is performed on the inner side surface of the inner-layer hard tube 101.

Preferably, in the present invention, the length of the woven mesh structure 102 obtained in step S2: toughened structural layer application is greater than the length of the inner-layer hard tube 101, and the length of the toughened structural layer 102 is the same as a length of the outer-layer tube obtained in step S3: outer tube combination.

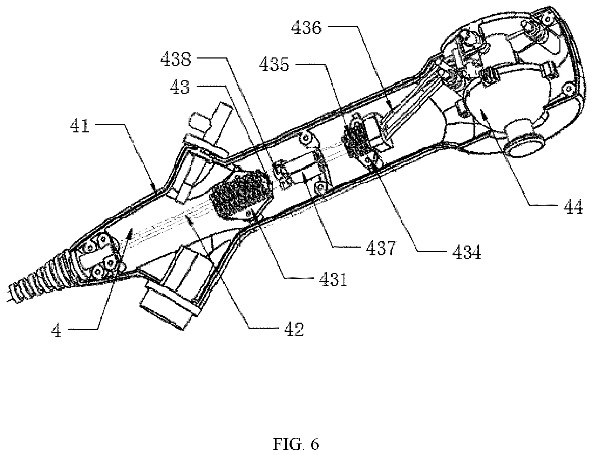

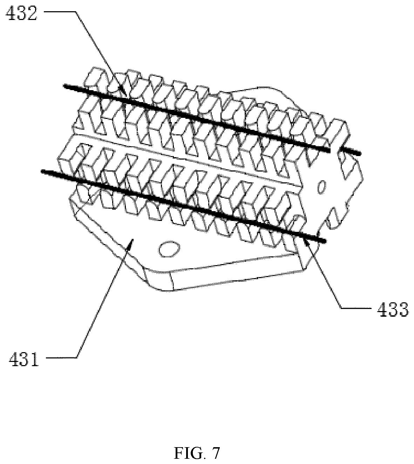

Referring to FIGs. 1-7, the present invention further provides a flexible joint driving device 4 of a disposable soft scope catheter. The flexible joint driving device 4 includes an operation handle housing 41, traction wires 42, a limiting buffer component 43, and a rear end control mechanism 44, wherein the traction wires 42, the limiting buffer component 43, and the rear end control mechanism 44 are all disposed inside the operation handle housing 41, one end of each of the traction wires 42 passes through the limiting buffer component 43 and is connected to the rear end control mechanism 44, and the other end of each of the traction wires 42 is connected to the snake bone structure 3.

The limiting buffer component 43 includes a first fixed seat 431, several first clamping slots 432, several buffer springs 433, a second fixed seat 434, second clamping slots 435, guide tubes 436, threaded sleeve tubes 437, and an adjusting bolt 438. The first fixed seat 431 is disposed on an inner side wall of the operation handle housing 41. The first clamping slots 432 are disposed on the first fixed seat 431. The buffer springs 433 are disposed on the first clamping slots 432. The traction wires 42 pass through interiors of the buffer springs 433. The second fixed seat 434 is disposed on the inner side wall of the operation handle housing 41. The second clamping slots 435 are disposed on the second fixed seat 434. The guide tubes 436 are disposed on the second clamping slots 435. The traction wires 42 pass through the guide tubes 436 and are connected to the rear end control mechanism 44. The threaded sleeve tubes 437 and the adjusting bolt 438 are disposed between the traction wires 42. The threaded sleeve tubes 437 are in a threaded connection with the adjusting bolt 438. The guide tubes 436 are provided with a plurality of steering deflection points.

The rear end control mechanism 44 includes a hook hinge that includes a base, a limiting ring, and an adjusting rod. Soft lock driving is divided into three major portions, so as to facilitate installation carried out by workers and reduce costs. Meanwhile, the soft lock driving mode in the present invention can satisfy different application situations of a soft joint.

In order to facilitate the installation carried out by the workers, the hook hinge is used to control a soft lock to drive a motion of the soft scope catheter with good stability. Driving by the traction wires 42 can enable the soft scope catheter to produce a multi-degree of freedom motion, thereby increasing flexibility of the soft scope catheter and satisfying different application situations of the soft scope catheter. The soft lock driving is divided into three major portions. The limiting buffer component 43 realizes an adjustment of each traction wire 42, thereby ensuring consistency in tensile forces of all the traction wires 42 while protecting the traction wires 42. Due to different rear end structure designs, there is a risk of entanglement and breakage of the traction wires 42 during a motion and a process of reaching the rear end control mechanism 44. In this case, the guide tubes 436 made of guide spring tubes or guide soft tubes may be selected to protect the traction wires 42, thereby increasing stability during the motion and a service life of the traction wires 42. Meanwhile, the limiting buffer component 43 also plays a role of guiding the traction wires 42. The guide tubes 436 guide each traction wire 42 to reach a desired position and to be connected to the rear end control mechanism 44, so as to ensure a stable state of each traction wire 42 during the driving process, thereby solving the problem of entanglement in the soft lock.

Experimental test: 100 disposable soft scope catheters processed by this process were selected and named A, and 100 disposable soft scope catheters processed by the conventional process were selected and named B. A diameter difference detection and a connection strength test were performed at a junction of the outer tube, and the following results were obtained:

**Table I**

| Group items | Average connection diameter difference (mm) | Anti-tensile rate % | Anti-aging rate % |
|---|---|---|---|
| A | 0.012 | 97.5 | 89.75 |
| B | 0.056 | 94.32 | 86.4 |

### Embodiment 2

Referring to FIGs. 1-5, the present invention provides the following technical solution: a disposable soft scope catheter includes a hard tube end 1 and a soft tube end 2. The hard tube end 1 includes an inner-layer hard tube 101, a toughened structural layer 102, and an outer-layer hard tube 103. The toughened structural layer 102 is disposed on an inner surface of the outer-layer hard tube 103, and the inner-layer hard tube 101 is disposed on an inner surface of the toughened structural layer 102.

The toughened structural layer 102 is either of a woven mesh structural layer and a spiral spring.

The soft tube end 2 includes an outer-layer soft tube 21 that is disposed at one end of the outer-layer hard tube 103. A snake bone structure 3 is distributed inside the outer-layer soft tube 21, and nested on an inner side wall of the toughened structural layer 102.

Preferably, a hardness of the inner-layer hard tube 101 is greater than a hardness of the outer-layer hard tube 103, the toughened structural layer 102 extends to an end of the outer-layer soft tube 21 away from the outer-layer hard tube 103, and a step structure is formed at an end of the inner-layer hard tube 101 close to the outer-layer soft tube 21.

Preferably, the snake bone structure 3 includes several snake bones. One end of the snake bone structure 3 abuts against an end face of the step structure of the inner-layer hard tube 101. A thickness of the snake bone structure 3 is less than a tube wall thickness of the inner-layer hard tube 101. An upper bending degree and a lower bending degree of the snake bone structure 3 are both greater than 280°.

Preferably, a linking structure of the snake bone structure 3 is molded integrally by laser-cutting a metal round tube, and includes a patterned rotation structure 31. The patterned rotation structure 31 includes first-stage rotation members and second-stage rotation members fitting with the first-stage rotation members, wherein the first-stage rotation members include first-stage arc-shaped slide blocks 3101 and second-stage arc-shaped slideways 3102, and the second-stage rotation members include first-stage arc-shaped slideways 3103 and second-stage arc-shaped slide blocks 3104. The first-stage arc-shaped slide blocks 3101 fit with the first-stage arc-shaped slideways 3103, the second-stage arc-shaped slide blocks 3104 fit with the second-stage arc-shaped slideways 3102, and the first-stage arc-shaped slide blocks 3101, the second-stage arc-shaped slideways 3102, the first-stage arc-shaped slideways 3103, and the second-stage arc-shaped slide blocks 3104 are concentric arcs.

Preferably, in the patterned rotation structure 31, the second-stage rotation members fitting with two ends of the first-stage rotation members are disposed vertically. When the metal round tube is in a straight line state, ends of the first-stage arc-shaped slide blocks 3101 and ends of the first-stage arc-shaped slideways 3103 form first-stage rotation avoidance clearances 32 and third-stage rotation avoidance clearances 33. Ends of the second-stage arc-shaped slide blocks 3104 and ends of the second-stage arc-shaped slideways 3102 form second-stage rotation avoidance clearances 34 and fourth-stage rotation avoidance clearances 35. The first-stage rotation avoidance clearances 32 and the second-stage rotation avoidance clearances 34 are disposed symmetrically with respect to both an X-axis and a Y-axis of a center of the concentric arcs.
he first-stage arc-shaped slide blocks 3101 perform an arc reciprocating motion along the first-stage arc-shaped slideways 3103 under the action of an external force, while the second-stage arc-shaped slide blocks 3104 perform an arc reciprocating motion along the second-stage arc-shaped slideways 3102, such that the first-stage rotation members and the second-stage rotation members form a relative bending state from an initial position.

The linking structure of the snake bone molded integrally by laser cutting solves the problem that the existing snake bone can only be achieved through riveting. As such, the snake bone is easy to process, and the product consistency is improved.

Preferably, the woven mesh structural layer includes any one of nylon, a metal, a fiber, and fluoroplastic.

The present invention further provides a fabrication method of a disposable soft scope catheter. The fabrication method of the disposable soft scope catheter in the present invention includes the following steps: S1: inner-layer hard tube fabrication; S2: toughened structural layer application; S3: outer tube combination; and S4: snake bone structure nesting, wherein:
S1: inner-layer hard tube fabrication includes: first fabricating the inner-layer hard tube 101 of a hard segment of the soft scope catheter by once molding, and performing a deburring treatment on two ends and outer and inner side surfaces of the inner-layer hard tube 101;
S2: toughened structural layer application includes: arranging the toughened structural layer 102 on an outer surface of the inner-layer hard tube 101 obtained in step S1: inner-layer hard tube fabrication, with a length of the toughened structural layer 102 being greater than a length of the inner-layer hard tube 101, such that a step is formed inside the hard segment of the catheter;
S3: outer tube combination includes: fabricating an outer-layer tube at once by combining the outer-layer hard tube 103 and the outer-layer soft tube 21, and adhering the outer-layer tube entirely in a wrapping manner to an outer surface of the toughened structural layer 102 obtained in step S2: toughened structural layer application; and
S4: snake bone structure nesting includes: nesting the snake bone structure 3 on the inner side wall of the toughened structural layer 102 inside the outer-layer soft tube obtained in step S3: outer tube combination, and abutting one end of the snake bone structure 3 against the step of the inner-layer hard tube 101.

Preferably, in the present invention, for the inner-layer hard tube 101 fabricated in step S1: inner-layer hard tube fabrication, prior to step S2: toughened structural layer application, abrasion treatment needs to be performed on the outer surface of the inner-layer hard tube 101, so as to ensure that the outer surface of the inner-layer hard tube 101 provides a friction force sufficient to better fix the toughened structural layer 102 on the outer surface of the inner-layer hard tube 101, while polishing treatment is performed on the inner side surface of the inner-layer hard tube 101.

Preferably, in the present invention, the length of the woven mesh structure 102 obtained in step S2: toughened structural layer application is greater than the length of the inner-layer hard tube 101, and the length of the toughened structural layer 102 is the same as a length of the outer-layer tube obtained in step S3: outer tube combination.

Referring to FIGs. 1-7, the present invention further provides a flexible joint driving device 4 of a disposable soft scope catheter. The flexible joint driving device 4 includes an operation handle housing 41, traction wires 42, a limiting buffer component 43, and a rear end control mechanism 44, wherein the traction wires 42, the limiting buffer component 43, and the rear end control mechanism 44 are all disposed inside the operation handle housing 41, one end of each of the traction wires 42 passes through the limiting buffer component 43 and is connected to the rear end control mechanism 44, and the other end of each of the traction wires 42 is connected to the snake bone structure 3.

The limiting buffer component 43 includes a first fixed seat 431, several first clamping slots 432, several buffer springs 433, a second fixed seat 434, second clamping slots 435, guide tubes 436, threaded sleeve tubes 437, and an adjusting bolt 438. The first fixed seat 431 is disposed on an inner side wall of the operation handle housing 41. The first clamping slots 432 are disposed on the first fixed seat 431. The buffer springs 433 are disposed on the first clamping slots 432. The traction wires 42 pass through interiors of the buffer springs 433. The second fixed seat 434 is disposed on the inner side wall of the operation handle housing 41. The second clamping slots 435 are disposed on the second fixed seat 434. The guide tubes 436 are disposed on the second clamping slots 435. The traction wires 42 pass through the guide tubes 436 and are connected to the rear end control mechanism 44. The threaded sleeve tubes 437 and the adjusting bolt 438 are disposed between the traction wires 42. The threaded sleeve tubes 437 are in a threaded connection with the adjusting bolt 438. The guide tubes 436 are provided with a plurality of steering deflection points.

The rear end control mechanism 44 includes a hook hinge that includes a base, a limiting ring, and an adjusting rod. Soft lock driving is divided into three major portions, so as to facilitate installation carried out by workers and reduce costs. Meanwhile, the soft lock driving mode in the present invention can satisfy different application situations of a soft joint.

In order to facilitate the installation carried out by the workers, the hook hinge is used to control a soft lock to drive a motion of the soft scope catheter with good stability. Driving by the traction wires 42 can enable the soft scope catheter to produce a multi-degree of freedom motion, thereby increasing flexibility of the soft scope catheter and satisfying different application situations of the soft scope catheter. The soft lock driving is divided into three major portions. The limiting buffer component 43 realizes an adjustment of each traction wire 42, thereby ensuring consistency in tensile forces of all the traction wires 42 while protecting the traction wires 42. Due to different rear end structure designs, there is a risk of entanglement and breakage of the traction wires 42 during a motion and a process of reaching the rear end control mechanism 44. In this case, the guide tubes 436 made of guide spring tubes or guide soft tubes may be selected to protect the traction wires 42, thereby increasing stability during the motion and a service life of the traction wires 42. Meanwhile, the limiting buffer component 43 also plays a role of guiding the traction wires 42. The guide tubes 436 guide each traction wire 42 to reach a desired position and to be connected to the rear end control mechanism 44, so as to ensure a stable state of each traction wire 42 during the driving process, thereby solving the problem of entanglement in the soft lock.

Experimental test: 100 disposable soft scope catheters processed by this process were selected and named A, and 100 disposable soft scope catheters processed by the conventional process were selected and named B. A diameter difference detection and a connection strength test were performed at a junction of the outer tube, and the following results were obtained:

**Table II**

| Group items | Average connection diameter difference (mm) | Anti-tensile rate % | Anti-aging rate % |
|---|---|---|---|
| A | 0.016 | 98.12 | 90.13 |
| B | 0.056 | 94.32 | 86.4 |

In present invention, with reference to Table I and Table II, the outer-layer tube can be fabricated by directly adhering and fixing the outer-layer hard tube 103 and the outer-layer soft tube 21 or fabricated by means of a gradual soft-hard change. The soft scope catheter is designed to be a layered structure. The hard tube end has a three-layer structure, wherein the inner-layer hard tube 101 has the highest hardness, the outer-layer hard tube 103 has the second highest hardness, and the toughened structural layer 102 is nested between the inner-layer hard tube 101 and the outer-layer hard tube 103. The soft tube end 2 has a two-layer structure including no inner tube layer, thereby facilitating one-step nesting of the snake bone structure 3 inside a front end of the soft tube. Moreover, the step formed between the toughened structural layer 102 and the inner-layer hard tube 101 of the hard tube end 1 inside the toughened structural layer 102 may be used for nesting, limiting and fixing the snake bone structure 3, thereby effectively improving reliability and flexibility of the snake bone. Through the once molding fabrication process of the soft scope catheter, the inner-layer hard tube 101 of the soft scope catheter is fabricated first, such that the toughened structural layer 102 of the hard tube end 1 extends to a length required by the entire soft scope catheter, thereby saving snake bone nesting and spot-welding procedures in an early phase of the conventional process. The integral woven steel wire mesh improves structural strength of the soft scope catheter greatly. The outer-layer tube can be fabricated by directly adhering and fixing the outer-layer hard tube 103 and the outer-layer soft tube 21 or fabricated by means of a gradual soft-hard change, thereby avoiding instability of segmental fixation followed by adhesion in the conventional process, while improving smoothness of an outer-layer surface of the soft scope catheter, and ensuring use comfort of patients during surgeries.

In addition, the adjustment of the tensile force of each traction wire 42 is achieved through linkage coordination of the limiting buffer component 43, thereby ensuring demands for the tensile forces of the traction wires 42 in different application situations. Meanwhile, the limiting buffer component 43 itself also plays a role of protecting the traction wires 42 and adjusting the positions of the traction wires 42.

The linking structure of the snake bone molded integrally by laser cutting solves the problem that the existing snake bone can only be achieved through riveting. As such, the snake bone is easy to process, and the product consistency is improved.

It should be noted that, the relational terms such as "first" and "second", etc. herein are merely used to distinguish one entity or operation from another entity or operation, and do not necessarily require or imply any actual relationship or order between these entities or operations. Moreover, the terms "include", "comprise", or any other variations thereof are intended to cover non-exclusive inclusions, such that a process, method, item, or device that includes a series of elements not only includes these elements, but also includes other elements that are not explicitly listed, or also includes elements inherent in such the process, method, item, or device.

Although the embodiments of the present invention have been illustrated and described, those of ordinary skills in the art may understand that multiple changes, modifications, substitutions, and variations may be made to these embodiments without departing from the principle and spirit of the present invention. The scope of the present invention is defined by the claims and equivalents thereof.

## Claims

1. A disposable soft scope catheter, comprising a hard tube end (1) and a soft tube end (2), **characterized in that**, the hard tube end (1) comprises an inner-layer hard tube (101), a toughened structural layer (102), and an outer-layer hard tube (103), the toughened structural layer (102) is dispose on an inner surface of the outer-layer hard tube (103), and the inner-layer hard tube (101) is disposed on an inner surface of the toughened structural layer (102);
the toughened structural layer (102) is either of a woven mesh structural layer and a spiral spring;
the soft tube end (2) comprises an outer-layer soft tube (21) that is disposed at one end of the outer-layer hard tube (103), and a snake bone structure (3) is disposed inside the outer-layer soft tube (21) and is disposed on an inner side wall of the toughened structural layer (102).

2. The disposable soft scope catheter of claim 1, **characterized in that**, a hardness of the inner-layer hard tube (101) is greater than a hardness of the outer-layer hard tube (103), the toughened structural layer (102) extends to an end of the outer-layer soft tube (21) away from the outer-layer hard tube (103), and a step structure is disposed at an end of the inner-layer hard tube (101) close to the outer-layer soft tube (21).

3. The disposable soft scope catheter of claim 2, **characterized in that**, the snake bone structure (3) comprises several snake bones, one end of the snake bone structure (3) is disposed on an end face of the step structure of the inner-layer hard tube (101), a thickness of the snake bone structure (3) is less than a tube wall thickness of the inner-layer hard tube (101), and an upper bending degree and a lower bending degree of the snake bone structure (3) are both greater than 280°.

4. The disposable soft scope catheter of claim 1, **characterized in that**, a linking structure of the snake bone structure (3) is molded integrally by laser-cutting a metal round tube, the linking structure of the snake bone structure (3) comprises a patterned rotation structure (31) that comprises first-stage rotation members and second-stage rotation members fitting with the first-stage rotation members, wherein the first-stage rotation members comprise first-stage arc-shaped slide blocks (3101) and second-stage arc-shaped slideways (3102), the second-stage rotation members comprise first-stage arc-shaped slideways (3103) and second-stage arc-shaped slide blocks (3104), the first-stage arc-shaped slide blocks (3101) fit with the first-stage arc-shaped slideways (3103), the second-stage arc-shaped slide blocks (3104) fit with the second-stage arc-shaped slideways (3102), and the first-stage arc-shaped slide blocks (3101), the second-stage arc-shaped slideways (3102), the first-stage arc-shaped slideways (3103), and the second-stage arc-shaped slide blocks (3104) are concentric arcs.

5. The disposable soft scope catheter of claim 4, **characterized in that**, in the patterned rotation structure (31), the second-stage rotation members fitting with two ends of the first-stage rotation members are disposed vertically; when the metal round tube is in a straight line state, ends of the first-stage arc-shaped slide blocks (3101) and ends of the first-stage arc-shaped slideways (3103) form first-stage rotation avoidance clearances (32) and third-stage rotation avoidance clearances (33), ends of the second-stage arc-shaped slide blocks (3104) and ends of the second-stage arc-shaped slideways (3102) form second-stage rotation avoidance clearances (34) and fourth-stage rotation avoidance clearances (35), and the first-stage rotation avoidance clearances (32) and the second-stage rotation avoidance clearances (34) are disposed symmetrically with respect to both an X-axis and a Y-axis of a center of the concentric arcs;
the first-stage arc-shaped slide blocks (3101) perform an arc reciprocating motion along the first-stage arc-shaped slideways (3103) under the action of an external force, while the second-stage arc-shaped slide blocks (3104) perform an arc reciprocating motion along the second-stage arc-shaped slideways (3102), such that the first-stage rotation members and the second-stage rotation members form a relative bending state from an initial position.

6. The disposable soft scope catheter of claim 1, **characterized in that**, the woven mesh structural layer includes any one of nylon, a metal, a fiber, and fluoroplastic.

7. A fabrication method of the disposable soft scope catheter of claim 1, **characterized by** comprising the following steps: S1: inner-layer hard tube fabrication; S2: toughened structural layer application; S3: outer tube combination; and S4: snake bone structure nesting, wherein:
S1: inner-layer hard tube fabrication comprises: first fabricating the inner-layer hard tube (101) of a hard segment of the soft scope catheter through once molding, and performing deburring treatment on two ends and outer and inner side surfaces of the inner-layer hard tube (101);
S2: toughened structural layer application comprises: arranging the toughened structural layer (102) on an outer surface of the inner-layer hard tube (101) obtained in step S1: inner-layer hard tube fabrication, with a length of the toughened structural layer (102) being greater than a length of the inner-layer hard tube (101), such that a step is formed inside the hard segment of the catheter;
S3: outer tube combination comprises: fabricating an outer-layer tube at once by combining the outer-layer hard tube (103) and the outer-layer soft tube (21), and adhering the outer-layer tube entirely in a wrapping manner to an outer surface of the toughened structural layer (102) obtained in step S2: toughened structural layer application; and
S4: snake bone structure nesting comprises: nesting the snake bone structure (3) on the inner side wall of the toughened structural layer (102) inside the outer-layer soft tube (21) obtained in step S3: outer tube combination, and abutting one end of the snake bone structure (3) against the step of the inner-layer hard tube (101).

8. The fabrication method of the disposable soft scope catheter of claim 7, **characterized in that**, for the inner-layer hard tube (101) obtained in step S1: inner-layer hard tube fabrication, prior to step S2: toughened structural layer application, abrasion treatment is required to be performed on the outer surface of the inner-layer hard tube (101), while polishing treatment is performed on the inner side surface of the inner-layer hard tube (101).

9. The fabrication method of the disposable soft scope catheter of claim 7, **characterized in that**, the length of the woven mesh structure (102) obtained in step S2: toughened structural layer application is greater than the length of the inner-layer hard tube (101), and the length of the toughened structural layer (102) is the same as a length of the outer-layer tube obtained in step S3: outer tube combination.

10. The disposable soft scope catheter of any one of claims 1-6, further comprising a flexible joint driving device (4), **characterized in that**, the flexible joint driving device (4) comprises an operation handle housing (41), traction wires (42), a limiting buffer component (43), and a rear end control mechanism (44), wherein the traction wires (42), the limiting buffer component (43), and the rear end control mechanism (44) are all disposed inside the operation handle housing (41), one end of each of the traction wires (42) passes through the limiting buffer component (43) and is connected to the rear end control mechanism (44), and the other end of each of the traction wires (42) is connected to the snake bone structure (3).
